# EUROPEAN PATENT SPECIFICATION

(11) **EP 2 595 553 B1**
(45) Date of publication and mention of the grant of the patent: **30.03.2016**
(21) Application number: 11810214.4
(22) Date of filing: 18.07.2011
(51) Int. Cl.: A61B 17/70, A61B 17/86

(54) **EXTENSIONS FOR SPINAL ANCHORS**
ERWEITERUNGEN FÜR WIRBELSÄULENANKER
EXTENSIONS POUR ANCRAGES VERTÉBRAUX

(30) Priority: 19.07.2010 US 838581
(43) Date of publication of application: 29.05.2013
(73) Proprietor: Warsaw Orthopedic, Inc., Warsaw, IN 46581 (US)
(72) Inventor: KAVE, Douglas D., Byhalia Mississippi 38611 (US)
(74) Representative: Viering, Jentschura & Partner mbB Patent- und Rechtsanwälte
(86) International application number: PCT/US2011/044312
(87) International publication number: WO 2012/012310

(56) References cited:
- US-A1- 2005 215 999
- US-A1- 2006 264 962
- US-A1- 2007 106 123
- US-A1- 2008 228 228
- US-A1- 2009 062 822
- US-A1- 2009 062 864
- US-B2- 7 179 261
- US-B2- 7 666 188

## Description

### BACKGROUND

Various devices and methods for stabilizing bone structures have been used for many years. For example, the fracture of an elongated bone, such as a femur or humerus, can be stabilized by securing a plate to the fractured bone across the fracture. The plate extends across the fractured area and thus stabilizes the fractured components of the bones relative to one another in a desired position. When the fracture heals, the plate can be removed or left in place, depending on the type of plate that is used.

Another type of stabilization technique uses one or more elongated rods extending between components of a bony structure and secured to the bony structure to stabilize the components relative to one another. The components of the bony structure are exposed and one or more bone engaging fasteners are placed into each component. The elongated rod is then secured to the bone engaging fasteners in order to stabilize the components of the bony structure.

One problem associated with the above described stabilization structures is that the skin and tissue surrounding the surgical site must be cut, removed, and/or repositioned in order for the surgeon to access the location where the stabilization device is to be installed. This repositioning of tissue causes trauma, damage, and scarring to the tissue. There are also risks that the tissue will become infected and that a long recovery time will be required after surgery for the tissue to heal.

Minimally invasive surgical techniques are particularly desirable in, for example, spinal and neurosurgical applications because of the need for access to locations deep within the body and the presence of vital intervening tissues. The development of percutaneous minimally invasive spinal procedures has yielded a major improvement in reducing recovery time and post-operative pain because they require minimal, if any, muscle dissection and can be performed under local anesthesia. These benefits of minimally invasive techniques have also found application in surgeries for other locations in the body where it is desirable to minimize tissue disruption and trauma. However, there remains a need for further improvements in instruments, systems and methods for stabilizing bony structures using minimally invasive techniques.

In this respect, United States Patent Application Publication US 2007/0106123A1 discloses a surgical retractor comprising: at least one elongate member; a coupling region disposed at one end of the surgical retractor, the coupling region having an opening located at a distal end thereof and at least one relief region; and a flexible joint coupling the elongate member to the coupling region. Further, United States Patent Application Publication US 2008/0228228A1 discloses a spinal stabilization apparatus, comprising: a vertebral anchor comprising a head portion and a bone attachment portion; a slot in said head portion, said slot having an open end opposite said bone attachment portion; an elongate flexible guide removably coupled to said head portion of said vertebral anchor and adapted to extend percutaneously from a surgical site; and a channel in said guide, said channel extending longitudinally of said guide and communicating with said slot through said open end. Further, United States Patent Application Publication US 2006/0264962A1 discloses a system for providing access to a spine of a patient, the system comprising: a first cannula comprising: a first blade; and a second blade discrete from the first blade; wherein the first and second blades are positionable substantially parallel to each other to provide the first cannula such that the first cannula has a distal end insertable into the patient to provide access to the spine, the distal end having a docking element discrete from and securable to a connecting element implantable in a first vertebra of the spine.

### SUMMARY

The invention provides a spinal surgical system according to claim 1. Further embodiments of the invention are described in the dependent claim. Any method or procedure described herein as such does not form part of the invention but is background art which is described to facilitate understanding of the invention.

Herein, there are described systems and methods for positioning a connecting member adjacent the spinal column that include at least one anchor assembly having an anchor engageable to bony structure and a receiver for receiving the connecting member. An extension is engaged to the receiver and defines a pathway that extends proximally from the receiver. The connecting member is movable along the extension to the receiver of the bone anchor. The extension is removable from the receiver of the bone anchor after the connecting member is positioned in the receiver to provide a low profile anchor assembly when the connecting member and bone anchor are finally implanted in the patient.

These and other aspects will be apparent from the following description of the illustrated embodiments.

### BRIEF DESCRIPTION OF THE FIGURES

FIG. 1 is a perspective view of an anchor assembly.
FIG. 2 is an enlarged portion of the perspective view of FIG. 1.
FIG. 3 is a top plan view of the anchor assembly of FIG. 1.
FIG. 4 is a longitudinal section view of the portion of the anchor assembly of FIG. 2.
FIG. 5 is a perspective view of the longitudinal section view of FIG. 5.
FIG. 6 is an elevation view of a portion of the anchor assembly showing advancement and guiding of a connecting member therein.
FIG. 7 is a diagrammatic elevation view of a method employing multiple anchor assemblies engaged to a spinal column to guide a connecting member in a minimally invasive procedure.
FIG. 8 is a diagrammatic elevation view of another embodiment method employing multiple anchor assemblies engaged to a spinal column to guide a connecting member in a minimally invasive procedure.
FIG. 9 is a diagrammatic elevation view showing the method of FIG. 8 with the connecting member positioned between anchors of the anchor assemblies.

### DESCRIPTION OF THE ILLUSTRATED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is thereby intended. Any such alterations and further modifications in the illustrated devices and described methods, and any such further applications of the principles of the invention as illustrated herein are contemplated as would normally occur to one skilled in the art to which the invention relates.

Anchor assemblies are provided that are engageable to a bony structure, such as one or more vertebrae of a spinal column, to guide placement of one or more connecting members from a location outside patient, or within the patient but remote from an implantation location, to or more adjacent to an implantation location in the patient. The connecting member can be an elongated spinal rod, tether, bar, plate, wire, or other suitable device that is to be engaged to one or more bone anchors. The anchor assemblies are particularly suited for minimally invasive surgical procedures, but are not restricted to such procedures. Furthermore, although its use and application is described with regard to spinal surgery, applications in surgeries other than spinal surgery are also contemplated.

In one form, surgical systems are provided that include at least one anchor assembly enagageable to a spinal column. The anchor assembly includes a bone anchor with a bone engaging portion and a receiver for receiving a connecting member. An extension is engaged to and extends from the receiver. The extension includes a pair of arms that define a space between the arms that extends proximally from a proximal end opening of the receiver. The connecting member is positionable into the space and movable along the arms through a proximal end opening of the receiver and into the receiver. The connecting member can then be secured to the bone anchor with an engaging member engaged to the receiver. The extension is removable from the receiver so that the anchor assembly has a low-profile in the patient post-surgery.

In another form, surgical systems are provided that include at least a pair of anchor assemblies engaged to the spinal column. The anchor assemblies each include an extension engaged to a bone anchor so that the extension extends proximally from the bone anchor. The anchor assemblies are implanted into the patient while the extensions extend proximally away from the implantation location. The extensions guide the placement of a connecting member from a position remote from implantation location to a position more adjacent the spinal column or to the implantation location. The extensions are configured so that when the connecting member is guided adjacent to the spinal column, the connecting member extends through the bone anchors of the anchor assemblies. The connecting member is secured to the bone anchor assemblies and provides stabilization of the spinal column segment to which the bone anchors assemblies are attached. The extensions are removed from the bone anchors after implantation of the connecting member so that the connecting member implantation and extension removal is accomplished without invasively accessing the patient's body.

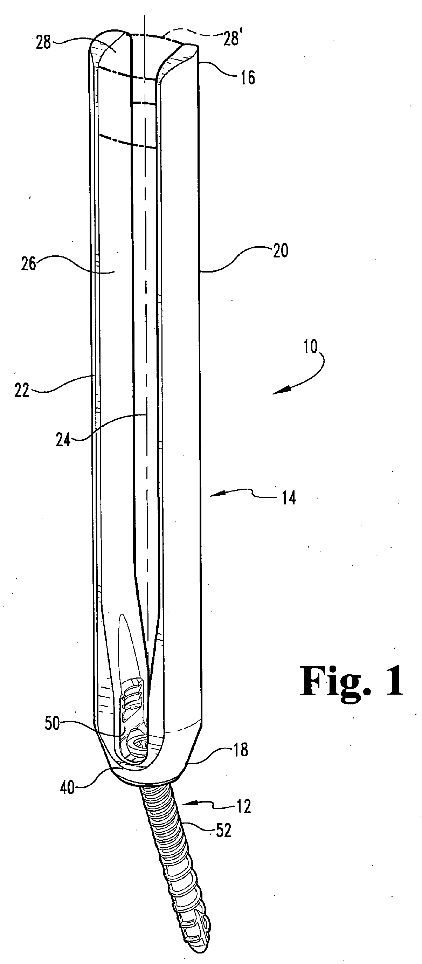

Referring to Fig. 1, one embodiment of an anchor assembly 10 is shown. Anchor assembly 10 includes a bone anchor 12 and an extension 14 extending proximally from bone anchor 12 to a proximal end 16. Extension 14 extends from proximal end 16 to a distal end 18 where it is secured to bone anchor 10 so that the bone anchor and extension form an anchor assembly that is implanted as a unit into the patient. Extension 14 includes elongate arms 20, 22 extending longitudinally on opposite sides of a central longitudinal axis 24 to proximal end 16. Elongate arms 20, 22 form a space 26 therebetween to receive the connecting member between arms 20, 22.

In one embodiment, arms 20, 22 include a length sufficient to locate proximal end 16 outside the skin and tissue of the patient when bone anchor 20 is secured to bony structure within the patient. In the illustrated embodiment, arms 20, 22 form a proximal opening 28 therebetween to allow the connecting member to be placed through the proximal end opening 28 into space 26 between arms 20, 22. In an alternative embodiment, a proximal portion 28' is provided that extends between and connects arms 20, 22 to close the proximal end opening, requiring the connecting member to be positioned into space 26 from the sides of arms 20, 22 with the connecting member oriented in a length-wise manner that is transverse to longitudinal axis 24.

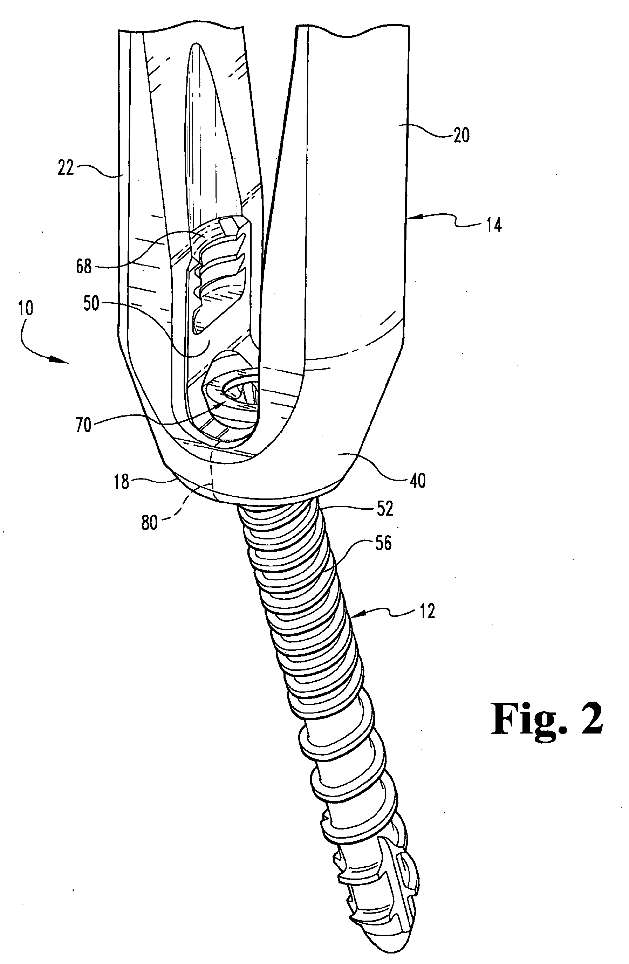

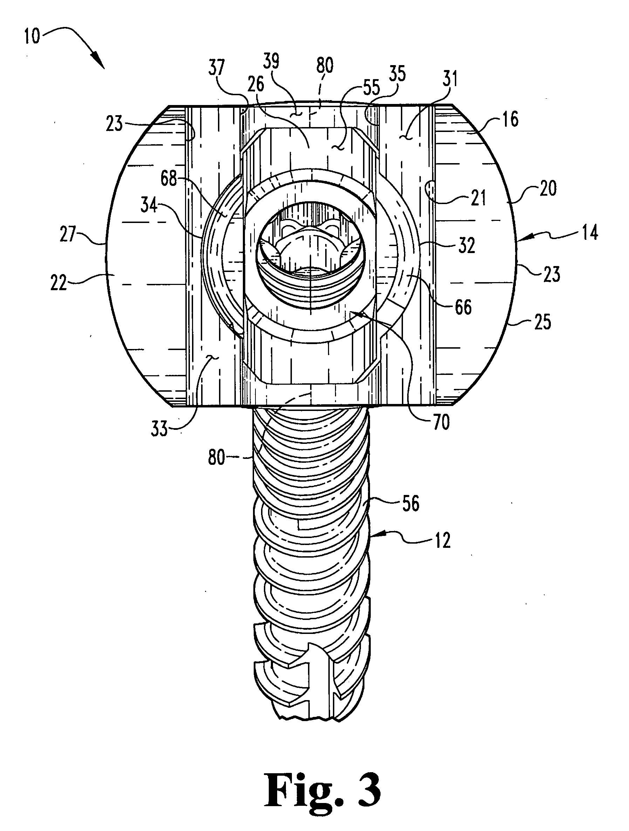

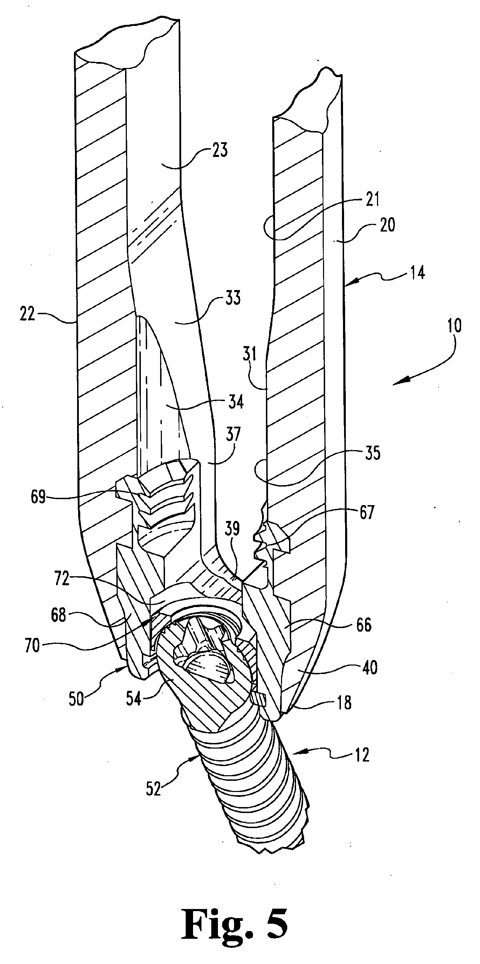

Referring further to Figs. 2-5, bone anchor 12 includes a proximal receiver 50 and a distal bone engaging portion 52. Receiver 50 receives the connecting member 100 from space 26 of extension 14. Extension 14 is molded, formed, cast, extruded, overlaid, coupled, glued, fastened, clamped, press-fit, or otherwise positioned around receiver 50 so that anchor assembly 10 is provided, at least initially to the surgeon, as a unit that includes anchor 12 and extension 14 for simultaneous implantation into the patient. When extension 14 is removed, bone anchor 12 is configured for post-operative implantation in the patient with connecting member 100 in receiver 50.

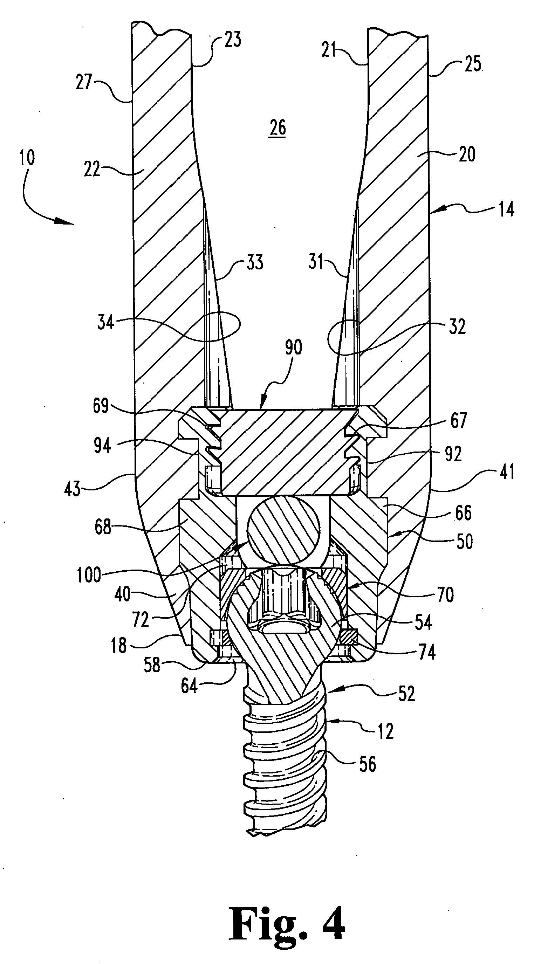

In the illustrated embodiment, receiver 50 forms a saddle that houses a portion of bone engaging member 52 and connecting member 100. Receiver 50 receives the connecting member 100 therethrough in an orthogonal or transverse orientation to longitudinal axis 24 and in an orientation that extends generally parallel with the spinal column. In one embodiment, connecting member 100 is an elongated spinal rod, and bone anchor 12 includes a bone screw portion extending from a distally facing end of receiver. The bone screw portion can be a multiaxial type screw pivotally received and carried by receiver 50 so that the receiver and bone screw are pivotal relative to one another. In another embodiment, the bone screw portion is non-pivotal or fixed relative to the receiver. Furthermore, connecting member 100 can be received in, on, or about the receiver 50 for engagement thereto with an engaging member 90. Engaging member 90 is shown in Fig. 4 as an externally threaded set screw. However, other embodiments contemplate engaging members that include one or more components in the form of a nut, cap, non-threaded member, friction fit member, twist-lock member, or combinations thereof that engage the receiver. Furthermore, the connecting member 100 can be rigid, semi-rigid, flexible, elastic, non-compression load bearing, or of other suitable form for extending between and stabilizing adjacent portions of the spinal column when secured thereto with one or more bone anchors.

In the illustrated embodiment, receiver 50 includes a pair of opposite side portions 66, 68 sized and spaced to accommodate connecting member 100 and engaging member 90 therebetween. Arms 20, 22 of extension 14 form an elongated extension of respective ones of the side portions 66, 68, and are joined therewith along the outer surface of the respective side portion 66, 68. Arms 20, 22 include a length extending proximally from side portions 66, 68 so that the proximal ends of arms 20, 22 are located outside the patient when anchor 12 is engaged to the spinal column. In one embodiment, this length is at least 30 millimeters. In another embodiment, the length of extensions 20, 22 is at least 50 millimeters. Other lengths are also contemplated. In one specific embodiment, the length is at least 100 millimeters, and extends about 120 millimeters from the anchor to the proximal end of the extension.

Side portions 66, 68 each include an internal thread profile 67, 69 to threadingly receive engaging member 90. Receiver 60 includes a hole 64 extending on longitudinal axis 24 that opens through a distally facing surface 58 of receiver 50. Hole 64 is sized and shaped to receive bone engaging portion 52 therethrough while supporting head 54 of bone engaging member 52 in receiver 50. Near the distally facing surface 58 at the bottom of receiver 50, hole 64 is surrounded by a retaining member 74. Retaining member 74 can be C-ring, washer, lip, or flange formed separately from or as an integral part of receiver 50 to support head 54 while allowing bone engaging member 52 to be positioned in any one of an infinite number of angular positions relative to receiver 50 and longitudinal axis 24. Other embodiments contemplate other engagement relationships between the bone engaging member 52 and receiver 50. In one embodiment, bone engaging member 52 is formed as a single, integral unit with receiver 50 and extends along longitudinal axis 24 in a co-axial arrangement. In another embodiment, bone engaging member 52 is captured in receiver 50 with a retaining member that allows pivotal movement relative to receiver 50 in a single plane or in a predetermined number of planes or directions relative to longitudinal axis 24.

In the particular illustrated embodiment of bone engaging member 52, it includes an initial configuration that allows pivoting movement in receiver 50 and is thereafter rigidly or semi-rigidly fixed in position when connecting member 100 is seated in receiver 50. Receiver 50 includes a crown 70 positioned on and around the proximal side of head 54. Crown 70 includes a proximal side that projects into a passage 72 defined between side portions 66, 68. When engaging member 90 is threadingly engaged to receiver 50, it pushes connecting member 100 in passage 72 against the proximal side of crown 70. Crown 70 is in turn pushed against the proximal side of head 54, which seats head 54 firmly against retaining member 74. The proximal side of head 54 may include a plurality of ridges or grooves that bite into a distally facing surface of crown 70 to enhance locking of bone engaging member 52 in position in receiver 50. In another embodiment, at least some motion between the receiver 50 and bone engaging member 52 is maintained by crown 70 when connecting member 100 is secured in receiver 50 with engaging member 90. Still other embodiments contemplate that crown 70 can be omitted and that connecting member 100 is seated directly against head 54 of bone engaging member 52 or against a bottom surface 55 of receiver 50 that extends on a distal side of passage 72 between side portions 66, 68.

Bone engaging portion 52 is shown as a bone screw with proximal head 54 and an elongated threaded shaft 56 extending distally from head 54 located in receiver 50. Other embodiments contemplate other forms for bone engaging member 52, such as a hook, post, tack, cerclage, staple, anchor, or other suitable bone engaging structure. Bone engaging member 52 can be a separate member that is connected with receiver 50, or formed as an integral, one-piece construct with receiver 50.

Extension 14 includes arms 20, 22 extending parallel to one another distally from proximal end 16 to a distal ring portion 40 that extends around receiver 50. Arms 20, 22 include planar facing surfaces 21, 23 that extend parallel to one another and parallel to longitudinal axis 24 that define space 26 therebetween. In addition, arms 20, 22 include outer, oppositely facing surfaces 25, 27, respectively, that define a convex curvature extending around longitudinal axis 24 and a generally linear profile paralleling longitudinal axis 24. The curved outer surfaces 25, 27 provide a smooth surface contour that holds back tissue from encroaching into space 26 while minimizing trauma to the tissue pressing against arms 20, 22.

Outer surfaces 25, 27 extend generally parallel to longitudinal axis 24 from proximal end 18 to a location 41, 43 of arms 20, 22 along the respective side portions 66, 68 of receiver 50 aligned proximally and distally with passage 72. Outer surfaces 25, 27 taper distally from these locations 41, 43 to ring portion 40. The tapering thickness of the wall of extension 14 continues to distal end 18. Extension 14 is positioned around receiver 50 so that most-distal part of distal end 18 is spaced proximally from the distally facing surface 58 of receiver 50 along the sides of side portions 66, 68. As explained further below, this facilitates removal of extension 14 from receiver 50, and allows pivoting of bone engaging portion 52 relative to receiver 50 without interference from extension 14.

Extension 14 includes ring portion 40 that extends completely around receiver 50 below the passage 72 of receiver 50. The wall thickness of ring portion 40 tapers distally to a minimum width at around ring portion 40. Extension 14 is made from a material with a thickness and/or material properties that allows arms 20, 22 of extension 14 to be separated from one another at a separation location, such as where ring portion 40 aligns with opening of passage 72 at the opposite sides of receiver 50. The surgeon applies sufficient force by twisting or pulling arms 20, 22 to sever arms 20, 22 at the minimum cross-section of extension 14 provided by ring portion 40 at the separation location. In one embodiment, ring portion 40 includes scoring 80, slits or other reduced wall thickness configuration at the separation locations. The scoring provides a separation region to facilitate splitting of ring portion 40 to allow arms 20, 22 to be separated from one another and from the respective side portion 66, 68.

Extension 14 also includes features to facilitate guiding and placement of connecting member 100 into passage 72 of receiver 50. Arms 20, 22 include a receiving portion along parallel surfaces 21, 23 that form a location to receive and provide initial guidance of the placement of connecting member 100 into and along space 26. Arms 20, 22 also include a tapered portion to direct connecting member 100 from its initial placement through space 26 into alignment with passage 72. Arm 20 includes a tapered surface portion 31 extending distally from inner surface 21 and arm 22 includes a tapered surface portion 33 extending distally from inner surface 23. Tapered surfaces portions 31, 33 converge toward one another and join with the respective alignment surface portions 35, 37, respectively, at or near the proximal end of receiver 50. Alignment surface portions 35, 37 extend parallel to one another to form an extension of inner surfaces of side portions 66, 68 defining passage 72 on opposite sides of receiver 50. Alignment surface portions 35, 37 are joined by concave connecting portion 39 that extends around and forms an extension of bottom surface 55 of receiver 50 on opposite sides of receiver 50.

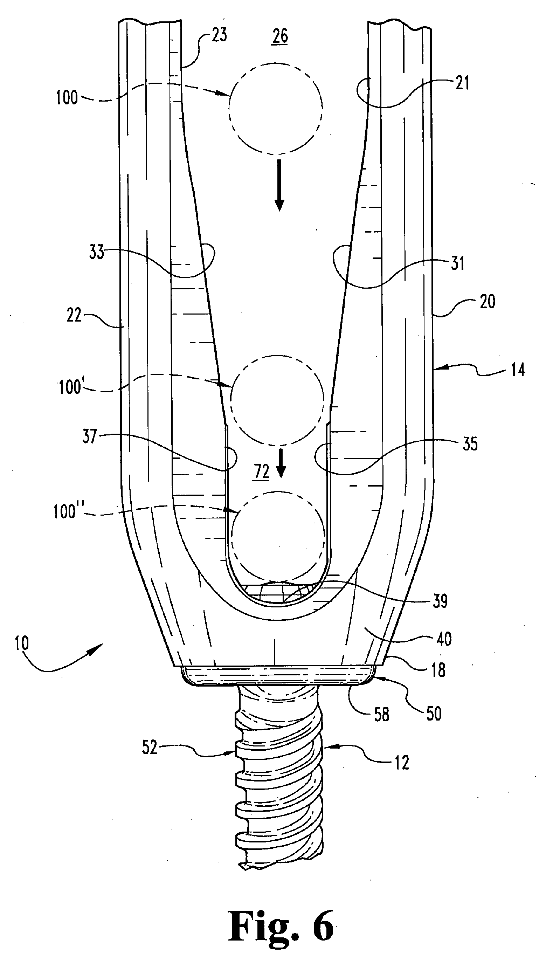

As shown in Fig. 6, connecting member 100 is initially positioned through space 26 of extension 14 at a location between inner surfaces 21, 23 at or near the proximal end 16 of extension 14. Since inner surfaces 21, 23 of arms 20, 22 are spaced farther apart than the alignment surface portions 35, 37, the surgeon has greater latitude in initially positioning connecting member 100 through extension 14. As connecting member 100 is advanced distally in extension 14 toward receiver 50, tapered surface portions 31, 33 center connecting member 100 in extension 14 and align it with passage 72 of receiver 50 as indicated by connecting member 100'. Alignment surface portions 35, 37 maintain and guide connecting member 100 into passage 72 as the connecting member is seated in receiver 50, as indicated by connecting member 100".

Extension 14 also includes a concave recess 32, 34 in respective ones of the tapered surface portions 31, 33. Recesses 32, 34 are concavely curved in a direction between the elongated sides of the respective arm 20, 22 so that the recess aligns with respective ones of side portions 66, 68. Recesses 32, 34 maintain the opening defined between the proximal ends of side portions 66, 68 through which connecting member 100 and engaging member 90 are received. This also allows alignment surfaces 35, 37 of arms 20, 22 to extend around the opposite sides of side portions 66, 68 where passage 72 opens and form an extension of passage 72 without interfering with placement of engaging member 90 through the proximal end opening of receiver 50.

In use, extension 14 is positioned around receiver 50 so that anchor assembly 10 is provided, at least initially to the surgeon, as a unit that includes anchor 12 and extension 14 for simultaneous implantation into the patient. Side portions 66, 68 can include respective ones of detents 92, 94 so that a portion of the respective arm portion 20, 22 extends therein to enhance the axial engagement of extension 14 to receiver 50. In one embodiment, extension 14 is made from a radiolucent material so that radiographic or fluoroscopic visualization of connecting member 100 is not obscured between arms 20, 22, allowing the surgeon to monitor advancement of connecting member 100 along extension 14 and through the tissue of the patient during the procedure. Examples of suitable materials for extension 14 include polyetheretherketone (PEEK), plastics, polymers, or aluminum, for example. Other materials are also contemplated, including radio-opaque materials and resorbable materials. Still other embodiments contemplate that extension 14 is made from a non-conductive material so that probes, taps, drivers and other instruments that employ electrical signals for neuro-stimulation are not shunted through extension 14 to the tissue around extension 14. Extension 14 may also include one or more holes, rockers, or other feature to allow attachment of a surgical reduction instrument to mechanically facilitate placement of connecting member 100 into passage 72 of receiver 50 and/or to align a vertebra attached to extension 14.

In yet other embodiments, at least a portion of extension 14 is made from a biocompatible resorbable material. For example, arms 20, 22 each include scoring, a reduced thickness arrangement, material weakness, or other removal feature that is located at or adjacent to the proximal end of the respective side portion 66, 68 of receiver 50. This allows the majority of the length of each arm 20, 22 to be removed from receiver 50, while the portion of extension 14 including ring portion 40 and the part of arms 20, 22 along the side portions 66, 68 remain engaged to receiver 50 post-operatively, i.e. after the surgical procedure is completed and the incision or opening into the patient is closed. At least these post-implantation parts of extension 14 can be made from a resorbable material to resorb post-operatively over time.

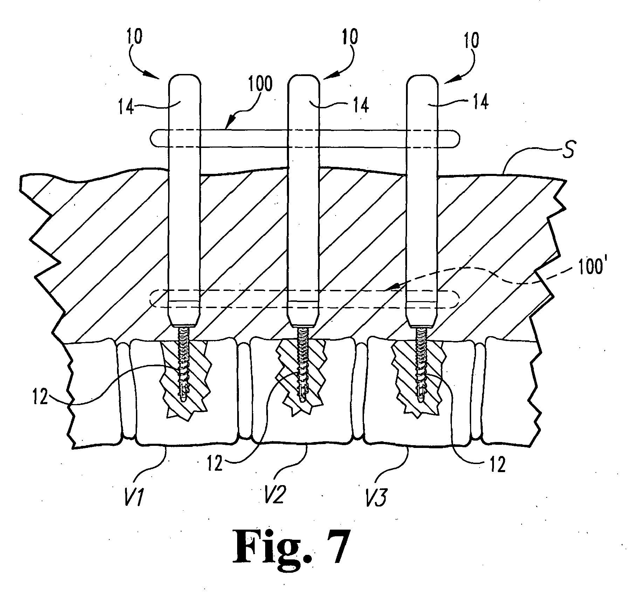

Referring to Fig. 7, there is shown another embodiment system employing multiple anchor assemblies 10 with bone anchors 12 engaged to respective ones of the vertebrae V1, V2, V3. Although three anchor assemblies 10 and vertebrae are shown, systems employing one, two, or four or more anchor extensions are also contemplated. Extensions 14 extend proximally from respective ones of the bone anchors 12 through the tissue of the patient and skin S to locate the proximal ends of extensions 14 outside the patient. Incisions are made to accommodate insertion of the anchors assemblies 10 and to extend between the extensions 14 to receive connecting member 100. Connecting member 100 is placed through extensions 14 outside the patient in a transverse orientation to extensions 14. Connecting member 100 is advanced along the extensions 14 and through skin S and the tissue to the implantation location adjacent anchors 12, as indicated by connecting member 100'. Extensions 14 also provide a pathway to allow placement of engaging members 90 therealong to secure connecting member 100' to the respective anchor 12.

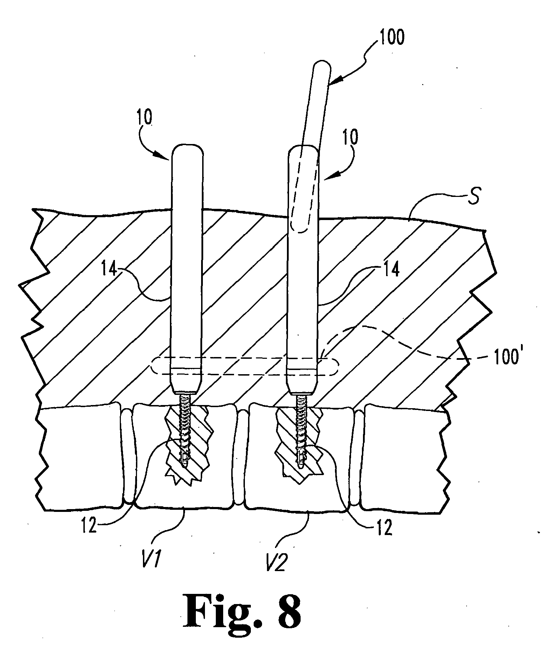

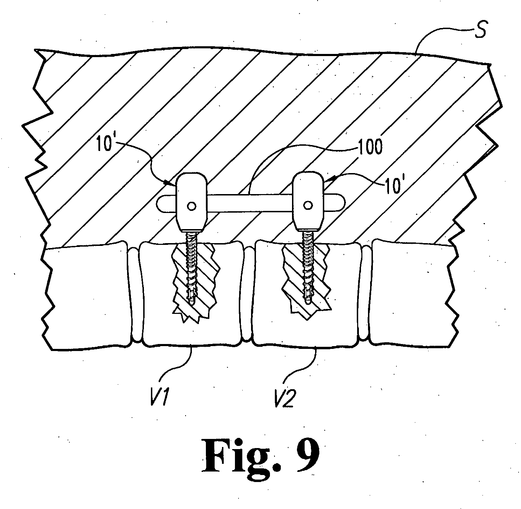

Fig. 8 shows an example of another embodiment system that employs two anchor assemblies 10 engaged to respective ones of two vertebrae V1 and V2. Connecting member 100 is placed into one of the anchor extensions 14 with the connecting member 100 oriented in generally parallel orientation to the longitudinal axis of extension 14. As connecting member 100 is advanced toward the vertebrae, connecting member 100 is pivoted from its initial orientation below skin S and tissue of the patient so that connecting member 100 extends from one of the bone anchors 12 to the other bone anchor 12, as indicated by connecting member 100'. In this procedure, an incision between anchor extensions 14 can be omitted. Other embodiments also contemplate that this procedure could be employed in procedures using one anchor assembly 10, or more than two anchor assemblies 10. As shown in Fig. 9, after connecting member 100 is implanted and engaged to the bone anchors, extensions 14 are removed from the bone anchors to leave modified anchor assemblies 10' for implantation in the patient.

## Claims

1. A spinal surgical system, comprising:
a connecting member (100) including an elongated body having a length sized to extend between at least two vertebrae (V1, V2, V3); and
an anchor assembly (10) including a distal bone engaging portion (52) engageable to bony structure and a receiver (50) extending proximally from said bone engaging portion (52), said receiver (50) including a passage (72) between opposite side portions (66, 68) of said receiver (50) with said passage (72) opening at opposite sides of said receiver (50) and with said passage further opening at a proximal end of said receiver (50), said anchor assembly (10) further including an extension (14) having a pair of elongated arms (20, 22) extending along a longitudinal axis (24) from a proximal end of said pair of arms (20, 22) to said side portions (66, 68) of said receiver (50), said extension (14) including a distal ring portion (40) connected with said pair of arms (20, 22) so that said ring portion (40) extends around said receiver (50) with said ring portion (40) located distally of said opposite side openings of said passage (72) through said receiver (50) and a distal end of said ring portion (40) located proximally of a distal facing surface (58) of said receiver (50) from which said bone engaging portion (52) extends, said extension (14) further including said pair of arms (20, 22) extending on an outer side of respective ones of said side portions (66, 68) of said receiver (50) so that said pair of arms (20, 22) and said ring portion (40) removably connect said extension (14) to an outer surface of said receiver (50), said pair of arms (20, 22) defining a space (26) therebetween that extends to said proximal opening (72) of said passage of said receiver (50), wherein said space (26) and said passage (72) are sized and shaped to receive said connecting member (100) therethrough so that connecting member (100) is movable from said space through said proximal end opening of said receiver (50) into said passage (72), wherein each of said pair of arms (20, 22) includes a planar inner surface (21, 23), said inner surfaces (21, 23) facing one another and extending parallel to one another and parallel to said longitudinal axis (24),
wherein said inner surfaces (21, 23) define a receiving portion adjacent said proximal end of said pair of arms (20, 22) where said inner surfaces (21, 23) are separated by a first distance; said inner surfaces (21, 23) define a tapered portion (31, 33) extending distally from said receiving portion where said inner surfaces converge toward one another to a proximal end of said receiver (50); and said inner surfaces (21, 23) define an alignment portion (35, 37) extending along and forming a parallel extension of said passage (72) of said receiver (50) along said opposite side openings of said passage (72) of said receiver (50), said inner surfaces (21, 23) being separated by a second distance along said alignment portion (35, 37) that is less than said first distance.

2. The system of claim 1, wherein said ring portion (40) includes a separation region where said ring portion (40) aligns with said opposite side openings of said passage (72) of said receiver (50), said extension (14) being separable at said separation region from said receiver (50) by separating said proximal end of said pair of arms (20, 22) from one another.

3. The system of claim 1, wherein each of said pair of arms (20, 22) includes an outer surface (25, 27) opposite said inner surface thereof that is convexly curved in a direction around said longitudinal axis (24).

4. The system of claim 1, wherein each of said inner surfaces includes a concavely curved recess (32, 34) in said tapered portion (31, 33) of said extension (14) and said recesses (32, 34) are aligned with and form a proximal extension of said proximal end opening of said passage (72) of said receiver (50).

5. The system of claim 1, wherein said distal ring portion (40) of said extension (14) tapers in thickness to a distal-most end of said extension (14).

6. The system of claim 5, wherein said ring portion (40) defines a separation location where said pair of arms (20, 22) are separable from one another by manipulating said proximal ends of said pair of arms (20, 22) away from one another, said separation location being aligned with said opposite side openings of said passage (72) of said receiver (50).

7. The system of claim 1, wherein said extension (14) is made from a radiolucent material molded with said receiver (50) so that said anchor assembly (10) is implantable in the patient as a single unit.

## Patentansprüche

1. Wirbelsäulenchirurgie-System aufweisend:
ein Verbindungsteil (100), welches einen langgestreckten Körper aufweist, der eine Länge hat, die dimensioniert ist, um sich zwischen mindestens zwei Wirbeln (V1, V2, V3) zu erstrecken, und
eine Ankereinrichtung (10), welche aufweist einen distalen Knochen-Eingriffsabschnitt (52), der mit Knochenstruktur in Eingriff gebracht werden kann, und einen Aufnehmer (50), der sich proximal von dem Knochen-Eingriffsabschnitt (52) erstreckt, wobei der Aufnehmer (50) einen Durchgang (72) zwischen gegenüberliegenden Seitenabschnitten (66, 68) des Aufnehmers (50) aufweist, wobei der Durchgang (72) an gegenüberliegenden Seiten des Aufnehmers (50) offen ist und wobei der Durchgang ferner an einem proximalen Ende des Aufnehmers (50) offen ist, wobei die Ankereinrichtung (10) ferner eine Verlängerung (14) aufweist, die ein Paar langgestreckter Arme (20, 22) aufweist, das sich entlang einer Längsachse (24) von einem proximalen Ende des Armpaares (20,22) zu den Seitenabschnitten (66, 68) des Aufnehmers (50) erstreckt, wobei die Verlängerung (14) einen distalen Ringabschnitt (40) aufweist, der mit dem Armpaar (20, 22) verbunden ist, sodass der Ringabschnitt (40) sich um den Aufnehmer (50) erstreckt, wobei der Ringabschnitt (40) distal von den gegenüberliegenden Seitenöffnungen des Durchgangs (72) durch den Aufnehmer (50) angeordnet ist und ein distales Ende des Ringabschnitts (40) proximal zu einer distalen StirnFläche (58) des Aufnehmers (50) angeordnet ist, von welcher sich der Knochen-Eingriffsabschnitt (52) erstreckt, wobei die Verlängerung (14) ferner aufweist, dass sich das Armpaar (20, 22) an einer Außenseite von jeweiligen der Seitenabschnitte (66, 68) des Aufnehmers (50) erstreckt, sodass das Armpaar (20, 22) und der Ringabschnitt (40) die Verlängerung (14) lösbar mit einer Außenfläche des Aufnehmers (50) verbinden, wobei das Armpaar (20, 22) einen Raum (26) dazwischen definiert, der sich zu der proximalen Öffnung (72) des Durchgangs des Aufnehmers (50) erstreckt, wobei der Raum (26) und der Durchgang (72) so dimensioniert und geformt sind, um das Verbindungsteil (100) dort hindurch aufzunehmen, sodass das Verbindungsteil (100) von dem Raum durch die proximale Endöffnung des Aufnehmers (50) in den Durchgang (72) hinein bewegbar ist, wobei jeder Arm des Armpaares (20, 22) eine ebene Innenfläche (21, 23) aufweist, wobei die Innenflächen (21, 23) einander zugewandt sind und sich parallel zueinander und parallel zu der Längsachse (24) erstrecken,
wobei die Innenflächen (21, 23) einen Aufnahmeabschnitt benachbart zu dem proximalen Ende des Armpaares (20, 22) definieren, wo die Innenflächen (21, 23) durch einen ersten Abstand separiert sind; wobei die Innenflächen (21, 23) einen angeschrägten Abschnitt (31, 33) definieren, der sich distal von dem Aufnahmeabschnitt erstreckt, wo die Innenflächen zu einem proximalen Ende des Aufnehmers (50) hin zusammenlaufen; und wobei die Innenflächen (21, 23) einen Ausrichtungsabschnitt (35, 37) definieren, der eine parallele Erweiterung des Durchgangs (72) des Aufnehmers (50) entlang der gegenüberliegenden Seitenöffnungen des Durchgangs (72) des Aufnehmers (50) bildet und sich dort entlang erstreckt, wobei die Innenflächen (21, 23) durch einen zweiten Abstand entlang des Ausrichtungsabschnitts (35, 37) separiert sind, der geringer ist als der erste Abstand.

2. Das System gemäß Anspruch 1, wobei der Ringabschnitt (40) einen Separierbereich aufweist, wo der Ringabschnitt (40) mit den gegenüberliegenden Seitenöffnungen des Durchgangs (72) des Aufnehmers (50) fluchtet, wobei die Verlängerung (14) an dem Separierbereich von dem Aufnehmer (50) separierbar ist, indem das proximale Ende des Armpaares (20, 22) voneinander separiert wird.

3. Das System gemäß Anspruch 1, wobei jeder Arm des Armpaares (20, 22) eine Außenfläche (25, 27) gegenüber der Innenfläche des Arms aufweist, die konvex in einer Richtung um die Längsachse (24) gekrümmt ist.

4. Das System gemäß Anspruch 1, wobei jede der Innenflächen eine konkav gekrümmte Aussparung (32, 34) in dem angeschrägten Abschnitt (31, 33) der Verlängerung (14) aufweist und die Aussparungen (32, 34) eine proximale Verlängerung der proximalen Endöffnung des Durchgangs (72) des Aufnehmers (50) bilden und mit dieser fluchten.

5. Das System gemäß Anspruch 1, wobei der distale Ringabschnitt (40) der Verlängerung (14) sich in der Dicke zu einem distalsten Ende der Verlängerung (14) hin verjüngt.

6. Das System gemäß Anspruch 5, wobei der Ringabschnitt (40) eine Separierstelle definiert, wo das Armpaar (20, 22) voneinander separierbar ist, indem die proximalen Enden des Armpaares (20, 22) voneinander weg manipuliert werden, wobei die Separierstelle mit den gegenüberliegenden Seitenöffnungen des Durchgangs (72) des Aufnehmers (50) fluchtet.

7. Das System gemäß Anspruch 1, wobei die Verlängerung (14) aus einem strahlendurchlässigen Material gemacht ist, das mit dem Aufnehmer (50) geformt ist, sodass die Ankereinrichtung (10) als eine einzelne Einheit in den Patienten implantierbar ist.

## Revendications

1. Système chirurgical vertébral comprenant :
un élément de raccordement (100) comprenant un corps allongé ayant une longueur dimensionnée pour s'étendre entre au moins deux vertèbres (V1, V2, V3) ; et
un ensemble d'ancrage (10) comprenant une partie de mise en prise osseuse distale (52) pouvant mettre en prise une structure osseuse et un récepteur (50) s'étendant de manière proximale à partir de ladite partie de mise en prise osseuse (52), ledit récepteur (50) comprenant un passage (72) entre des parties latérales opposées (66, 68) dudit récepteur (50) avec ledit passage (72) qui s'ouvre sur les côtés opposés dudit récepteur (50) et avec ledit passage qui s'ouvre en outre au niveau d'une extrémité proximale dudit récepteur (50), ledit ensemble d'ancrage (10) comprenant en outre une extension (14) ayant une paire de bras allongés (20, 22) s'étendant le long d'un axe longitudinal (24) à partir d'une extrémité proximale de ladite paire de bras (20, 22) vers lesdites parties latérales (66, 68) dudit récepteur (50), ladite extension (14) comprenant une partie annulaire distale (40) raccordée avec ladite paire de bras (20, 22) de sorte que ladite partie annulaire (40) s'étend autour dudit récepteur (50) avec ladite partie annulaire (40) positionnée de manière distale par rapport auxdites ouvertures latérales opposées dudit passage (72) à travers ledit récepteur (50) et une extrémité distale de ladite partie annulaire (40) positionnée de manière proximale par rapport à une surface de face distale (58) dudit récepteur (50) à partir de laquelle ladite partie de mise en prise osseuse (52) s'étend, ladite extension (14) comprenant en outre ladite paire de bras (20, 22) s'étendant sur un côté externe des parties respectives desdites parties latérales (66, 68) dudit récepteur (50) de sorte que ladite paire de bras (20, 22) et ladite partie annulaire (40) raccordent, de manière amovible, ladite extension (14) à une surface externe dudit récepteur (50), ladite paire de bras (20, 22) définissant un espace (26) entre eux qui s'étend vers ladite ouverture proximale (72) dudit passage dudit récepteur (50), dans lequel ledit espace (26) et ledit passage (72) sont dimensionnés et formés pour recevoir ledit élément de raccordement (100) à travers ces derniers de sorte que l'élément de raccordement (100) est mobile par rapport audit espace à travers ladite ouverture d'extrémité proximale dudit récepteur (50) dans ledit passage (72), dans lequel chacun de ladite paire de bras (20, 22) comprend une surface interne plane (21, 23), lesdites surfaces internes (21, 23) se faisant face et s'étendant parallèlement entre elles et parallèlement audit axe longitudinal (24),
dans lequel lesdites surfaces internes (21, 23) définissent une partie de réception adjacente à ladite extrémité proximale de ladite paire de bras (20, 22) où lesdites surfaces internes (21, 23) sont séparées par une première distance ; lesdites surfaces internes (21, 23) définissent une partie progressivement rétrécie (31, 33) s'étendant de manière distale à partir de ladite partie de réception où lesdites surfaces internes convergent l'une vers l'autre vers une extrémité proximale dudit récepteur (50) ; et lesdites surfaces internes (21, 23) définissent une partie d'alignement (35, 37) s'étendant le long de et formant une extension parallèle dudit passage (72) dudit récepteur (50) le long desdites ouvertures latérales opposées dudit passage (72) dudit récepteur (50), lesdites surfaces internes (21, 23) étant séparées par une seconde distance le long de ladite partie d'alignement (35, 37) qui est inférieure à ladite première distance.

2. Système selon la revendication 1, dans lequel ladite partie annulaire (40) comprend une région de séparation où ladite partie annulaire (40) s'aligne avec lesdites ouvertures latérales opposées dudit passage (72) dudit récepteur (50), ladite extension (14) pouvant être séparée, au niveau de ladite région de séparation, dudit récepteur (50) en séparant ladite extrémité proximale de ladite paire de bras (20, 22) l'un de l'autre.

3. Système selon la revendication 1, dans lequel chacun de ladite paire de bras (20, 22) comprend une surface externe (25, 27) opposée à ladite surface interne de ce dernier qui est incurvée de manière convexe dans une direction autour dudit axe longitudinal (24).

4. Système selon la revendication 1, dans lequel chacune desdites surfaces internes comprend un évidement (32, 34) incurvé de manière concave dans ladite partie progressivement rétrécie (31, 33) de ladite extension (14) et lesdits évidements (32, 34) sont alignés avec et forment une extension proximale de ladite ouverture d'extrémité proximale dudit passage (72) dudit récepteur (50).

5. Système selon la revendication 1, dans lequel ladite partie annulaire distale (40) de ladite extension (14) se rétrécit progressivement en épaisseur jusqu'à l'extrémité la plus distale de ladite extension (14).

6. Système selon la revendication 5, dans lequel ladite partie annulaire (40) définit un emplacement de séparation où ladite paire de bras (20, 22) sont séparables l'un de l'autre en éloignant lesdites extrémités proximales de ladite paire de bras (20, 22) l'une de l'autre, ledit emplacement de séparation étant aligné avec lesdites ouvertures latérales opposées dudit passage (72) dudit récepteur (50).

7. Système selon la revendication 1, dans lequel ladite extension (14) est réalisée à partir d'un matériau radiotransparent moulé avec ledit récepteur (50) de sorte que ledit ensemble d'ancrage (10) est implantable dans le patient sous la forme d'une unité unique.
